# EUROPEAN PATENT APPLICATION

(11) **EP 4 628 026 A1**
(43) Date of publication of application: **08.10.2025**
(21) Application number: 23896706.1
(22) Date of filing: 27.11.2023
(51) Int. Cl.: A61B 10/02, A61B 17/32, H02K 7/116

(54) **CONTROL METHOD FOR BIOPSY SAMPLING DEVICE, AND BIOPSY SAMPLING DEVICE**

(30) Priority: 30.11.2022 CN 202211529700
(71) Applicant: Chongqing Xishan Science & Technology Co., Ltd., Chongqing 401121 (CN)
(72) Inventor: GUO, Yijun, Chongqing 401121 (CN); CAI, Li, Chongqing 401121 (CN); TANG, Yirui, Chongqing 401121 (CN)
(74) Representative: Hoffmann Eitle
(86) International application number: PCT/CN2023/134218
(87) International publication number: WO 2024/114555

(57) **Abstract**

Disclosed are a biopsy sampling device and a control method therefor. The biopsy sampling device includes an outer cutter tube (21), an inner cutter tube (22), a first motor (11), and a second motor (12). The outer cutter tube (21) sleeves the inner cutter tube (22). A sampling window (21a) is formed in a side of a front end of the outer cutter tube (21). The first motor (11) is configured to drive the inner cutter tube (22) to move in an axial direction, and the second motor (12) is configured to drive the inner cutter tube (22) to rotate around an axis of the inner cutter tube. The control method includes: obtaining a sampling instruction, and according to the sampling instruction, controlling operating states of the first motor (11) and the second motor (12) to drive the inner cutter tube (22) to move relative to the outer cutter tube (21), so as to perform two or more samplings. During each sampling, first controlling the first motor (11) to rotate, so as to drive the inner cutter tube (22) to move in the axial direction to a specified sampling position; and then controlling the first motor (11) and the second motor (12) to simultaneously rotate, so as to drive the inner cutter tube (22) to move forward and rotate until the inner cutter tube (22) reaches a position where the sampling window is closed. During two consecutive samplings, directions of rotation of the inner cutter tube (22) around the axis of the inner cutter tube are opposite.

## Description

### RELATED APPLICATIONS

This application claims priority to Chinese patent application No. 202211529700.0, entitled "Control Method for Biopsy Sampling Device, and Biopsy Sampling Device", filed on November 30, 2022, the entire contents of which are incorporated by reference.

### TECHNICAL FIELD

The present application relates to the field of medical instrument technologies, and in particular, to a control method for a biopsy sampling device, and a biopsy sampling device.

### BACKGROUND

A biopsy is a technology of taking out a lesion tissue from the body of a patient by means of cutting, clamping, puncturing, or the like, for a pathological examination according to requirements of diagnosis, treatment, or the like. Biopsy sampling devices are configured to sample a living tissue from the human body. A widely used biopsy sampling device is the one that performs sampling through rotational cutting, and the device generally includes a cutter and a handle. The cutter includes an inner cutter tube and an outer cutter tube sleeving the inner cutter tube. A front end of the outer cutter tube is a tip for puncturing, and a side of the outer cutter tube close to the front end is provided with a sampling slot. A cutting edge is arranged at a front end of the inner cutter tube. The inner cutter tube is located at a foremost position to occlude the sampling slot during the puncturing. The inner cutter tube moves rearward after the tip reaches a specific position to expose the sampling slot, the tissue is sucked into the sampling slot under negative pressure, and in this case, the inner cutter tube moves forward and performs rotary cutting, and the tissue entering the sampling slot is cut off and accommodated in the front end of the inner cutter tube.

Currently, most biopsy needles include outer cutter tubes and inner cutter tubes movably arranged in the outer cutter tubes, and by a system, the inner cutter tubes are controlled to move forward and rearward along axes of the outer cutter tubes and meanwhile controlled to rotate along a fixed direction. Usually, a lot of samplings may be performed in one time of puncturing, and since the inner cutter tube rotates along one direction during the multiple samplings, and a side of a front portion of the inner cutter tube is provided with a negative pressure air inlet, the sampled tissue is prone to entangle the inner cutter tube through the air inlet, torsion of the inner cutter tube is increased, the system prompts overloading of a motor, and the tissue sampling cannot be completed.

### SUMMARY

In view of the above-mentioned deficiencies of the prior art, an object of the present application is to provide a control method for a biopsy sampling device, which prevents the inner cutter tube from being entangled by the tissue during the sampling.

In order to achieve the above object and other related objects, the technical solution of the present application is as follows.

Disclosed is a control method for a biopsy sampling device, the biopsy sampling device including an outer cutter tube and an inner cutter tube, the outer cutter tube sleeving the inner cutter tube, a sampling window being arranged in a side of a front end of the outer cutter tube, and the control method including:
obtaining a sampling instruction, the sampling instruction being configured for controlling operating states of a first motor and a second motor to drive the inner cutter tube to move relative to the outer cutter tube, so as to perform two or more samplings;
controlling the first motor to rotate according to the control instruction, so as to drive the inner cutter tube to move in an axial direction to a specified sampling position; and
controlling the first motor and the second motor to simultaneously rotate according to the control instruction, so as to drive the inner cutter tube to move forward and rotate until the inner cutter tube reaches a position where the inner cutter tube closes the sampling window;
where the first motor is configured to drive the inner cutter tube to move in the axial direction, and the second motor is configured to drive the inner cutter tube to rotate around an axis of the inner cutter tube. Directions of rotation of the inner cutter tube around the axis of the inner cutter tube are opposite in two consecutive samplings.

Optionally, the control method for a biopsy sampling device further includes: during the two consecutive samplings,
during the previous sampling, controlling the first motor to rotate to cause the inner cutter tube to move rearward from the position where the inner cutter tube closes the sampling window to a first sampling position, and after the inner cutter tube reaches the first sampling position, controlling the first motor and the second motor to rotate to cause the inner cutter tube to move forward and meanwhile rotate along a first direction until the inner cutter tube reaches the position where the inner cutter tube closes the sampling window;
during the next sampling, controlling the first motor to rotate to cause the inner cutter tube to move rearward to a specified second sampling position, and after the inner cutter tube reaches the second sampling position, controlling the first motor and the second motor to rotate to cause the inner cutter tube to move forward and rotate along a second direction until the inner cutter tube reaches the position where the inner cutter tube closes the sampling window;
where the first direction is opposite to the second direction, and rotation directions of the second motor during the two consecutive samplings are opposite.

Optionally, one sampling is performed every time the sampling instruction is received; or two or more consecutive samplings are performed every time the sampling instruction is received.

Optionally, during each sampling, when a first sampling instruction is received, the first motor is controlled to rotate to cause the inner cutter tube to move rearward to the specified second sampling position; and after the inner cutter tube reaches the second sampling position and a second sampling instruction is received, the first motor and the second motor are controlled to rotate simultaneously to cause the inner cutter tube to move forward and rotate along the second direction until the inner cutter tube reaches the position where the inner cutter tube closes the sampling window.

The present application further provides a biopsy sampling device, including an outer cutter tube and an inner cutter tube, the outer cutter tube sleeving the inner cutter tube, a sampling window being arranged in a side of a front end of the outer cutter tube, and the biopsy sampling device further including:
a first motor, the first motor being connected to the inner cutter tube through a first transmission mechanism to drive the inner cutter tube to move along an axial direction;
a second motor, the second motor being connected to the inner cutter tube through a second transmission mechanism to drive the inner cutter tube to rotate around an axis thereof; and
a controller respectively connected to the first motor and the second motor and configured to obtain a sampling instruction and control operating states of the first motor and the second motor according to the sampling instruction to drive the inner cutter tube to move relative to the outer cutter tube, so as to perform two or more samplings;
where the controller is further configured to:
   control the first motor to rotate according to the sampling instruction, so as to drive the inner cutter tube to move in the axial direction to a specified sampling position; and
   control the first motor and the second motor to simultaneously rotate according to the sampling instruction, so as to drive the inner cutter tube to move forward and rotate until the inner cutter tube reaches a position where the inner cutter tube closes the sampling window, thereby completing the sampling;
   where the first motor is configured to drive the inner cutter tube to move in the axial direction, and the second motor is configured to drive the inner cutter tube to rotate around the axis of the inner cutter tube; during two consecutive samplings, directions of rotation of the inner cutter tube around the axis of the inner cutter tube are opposite.

Optionally, the first transmission mechanism includes a reciprocating driving gear and a reciprocating driven gear that are meshed with each other, the first transmission mechanism further includes a first transmission component and a second transmission component sleeved by threads, the reciprocating driving gear being connected to an output end of the first motor, the reciprocating driven gear being mounted on the first transmission component and driving the first transmission component to rotate synchronously, and the second transmission component and the inner cutter tube being connected in a mode of being axially relatively fixed and circumferentially relatively rotatable.

Optionally, the biopsy sampling device further includes a handle housing, a first limiting structure for limiting axial movement of the first transmission component and a second limiting structure for limiting circumferential rotation of the second transmission component being arranged on the handle housing.

Optionally, a fourth transmission component sleeves the inner cutter tube, the fourth transmission component is axially and relatively fixedly connected to the inner cutter tube, an engaging groove is formed in an outer wall of the fourth transmission component along a circumferential direction, the second transmission component sleeves the fourth transmission component, an engaging portion extending into the engaging groove is formed in the second transmission component, and the second transmission component drives the fourth transmission component and the inner cutter tube to move axially through the engaging portion and the engaging groove.

Optionally, the second transmission mechanism includes a third transmission component, as well as a rotary cutting driving gear and a rotary cutting driven gear that are meshed with each other, the rotary cutting driving gear is connected to an output end of the second motor, the rotary cutting driven gear is mounted on the third transmission component and drives the third component to rotate synchronously, the third transmission component sleeves the inner cutter tube, and the third transmission component and the inner cutter tube are fitted in a mode of being axially and relatively movable and circumferentially and relatively fixed.

Optionally, the third transmission component sleeves the inner cutter tube, a groove is formed in an inner wall of the third transmission component along the axial direction, a protrusion portion fitting the groove is fixedly arranged on the outer cutter tube, and the protrusion portion and the groove are capable of moving relative to each other in the axial direction.

Optionally, the biopsy sampling device further includes an intermediate shaft, as well as a rotary cutting intermediate gear and a reciprocating intermediate gear that are mounted on the intermediate shaft, the first motor and the second motor being arranged side by side, the intermediate shaft being arranged in parallel between the first motor and the second motor, the rotary cutting intermediate gear being meshed with the rotary cutting driving gear and the rotary cutting driven gear simultaneously, and the reciprocating intermediate gear being meshed with the reciprocating driving gear and the reciprocating driven gear simultaneously.

As described above, the present application has the following beneficial effects: the double motors are configured in the biopsy handle of the biopsy sampling device, one motor is configured to control the inner cutter tube to move forward and rearward along the axial direction of the outer cutter tube, and the other motor is configured to control the inner cutter tube to rotate around the axis thereof during forward movement. Since the inner cutter tube does not rotate during rearward movement, the inner cutter tube rotates in different directions in two consecutive samplings during forward movement. In this way, the tissue entangled in the previous sampling can be released through reverse rotation in the next sampling, thereby effectively alleviating tissue entanglement, reducing a system fault rate, and improving an operation efficiency.

### BRIEF DESCRIPTION OF THE DRAWINGS

FIG. 1 is a schematic structural diagram of a biopsy sampling device according to an embodiment of the present application.
FIG. 2 is a schematic structural diagram of an outer cutter tube and an inner cutter tube in an embodiment of the present application.
FIG. 3 is a schematic structural diagram in which a part of a sampling window is exposed/closed by the inner cutter tube in an embodiment of the present application.
FIG. 4 is a partial sectional view (front view) of the biopsy sampling device according to an embodiment of the present application.
FIG. 5 is an enlarged view of a transmission part in FIG. 4.
FIG. 6 is a partial sectional view (top view) of the biopsy sampling device according to an embodiment of the present application.
FIG. 7 is a partial enlarged view of FIG. 6.
FIG. 8 is a schematic structural diagram of a third transmission component in an embodiment of the present application.
FIG. 9 is a sectional view of the third transmission component in an embodiment of the present application.
FIG. 10 is a schematic structural diagram of a fourth transmission component in an embodiment of the present application.
FIG. 11 is a flow chart of a control method for a biopsy sampling device according to an embodiment of the present application.
FIG. 12 is a flow chart of sampling control of the biopsy sampling device in an embodiment of the present application.
FIG. 13 is a flow chart of the sampling control of the biopsy sampling device in another embodiment of the present application.

### Reference signs

1: handle; 11: first motor; 12: second motor; 13: bracket; 14: intermediate shaft; 2: cutter assembly; 21: outer cutter tube; 21a: sampling window; 22: inner cutter tube; 31: reciprocating driving gear; 32: reciprocating intermediate gear; 33: reciprocating driven gear; 34: first transmission component; 35: second transmission component; 35a: engaging portion; 41: rotary cutting driving gear; 42: rotary cutting intermediate gear; 43: rotary cutting driven gear; 44: third transmission component; 44a: groove; 45: fourth transmission component; 45a: engaging groove; 45b: protrusion portion.

### DETAILED DESCRIPTION

To facilitate an understanding of the present application, the present application is described more fully hereinafter with reference to the accompanying drawings. Embodiments of the present application are shown in the drawings. However, the present application may be implemented in many different forms and is not limited to the embodiments set forth herein. Rather, these embodiments are provided to make the disclosure of the present application more thorough and complete.

Unless defined otherwise, all technical and scientific terms used herein have the same meanings as are commonly understood by those skilled in the art. The terms used herein in the specification of the present application are for the purpose of describing specific embodiments only but not intended to limit the present application.

The implementations of the present application are described below with reference to specific embodiments, and other advantages and effects of the present application will be readily apparent to those skilled in the art from the disclosure of the specification.

The front and rear orientations described in the present specification refer to the use state of a biopsy sampling device, and the side facing a patient is the front side and the side facing away from the patient is the rear side in use.

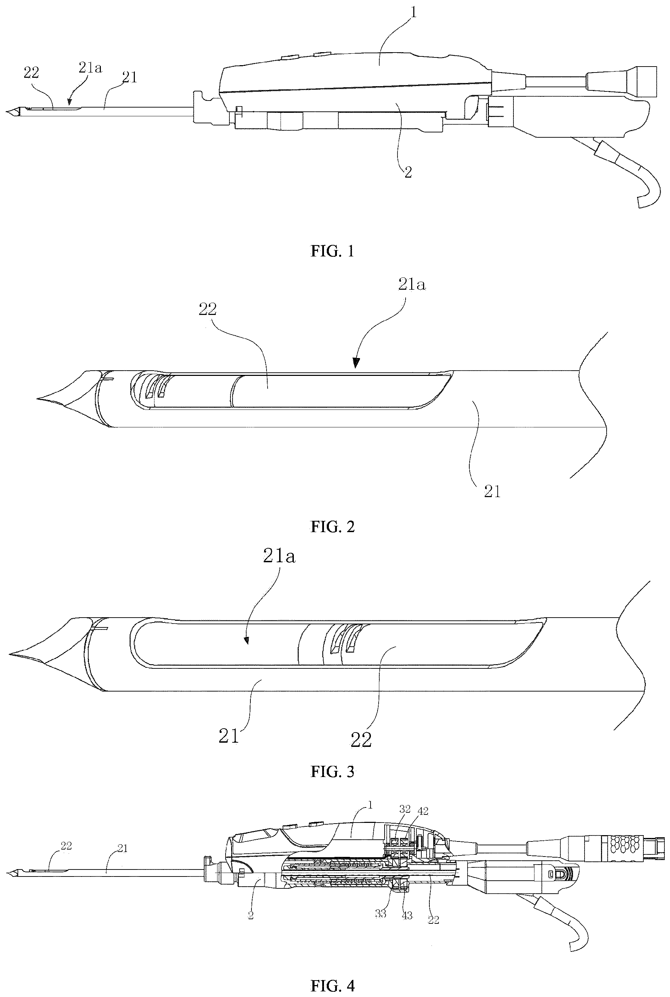

As shown in FIG. 1 to FIG. 6, embodiments of the present application provide a biopsy sampling device, including a handle 1 and a cutter assembly 2 connected to each other. The cutter assembly 2 includes an outer cutter tube 21 and an inner cutter tube 22 which are coaxially arranged. A front end of the outer cutter tube 21 is provided with a puncture tip, and a side of the outer cutter tube 21 close to the front end is provided with a sampling window 21a. A front end of the inner cutter tube 22 is provided with a cutting edge. The inner cutter tube 22 is sleeved with the outer cutter tube 21, and the inner cutter tube 22 can axially move relative to the outer cutter tube 21.

A first motor 11 and a second motor 12 for providing power are arranged in the handle 1. The first motor 11 is connected to the inner cutter tube 22 through a first transmission mechanism, and drives the inner cutter tube 22 to move in an axial direction (front-rear direction), so that the inner cutter tube 22 can move between a position where the inner cutter tube closes the sampling window and a position where the opening length of the sampling window is maximum.

The second motor 12 is connected to the inner cutter tube 22 through a second transmission mechanism, so as to drive the inner cutter tube 22 to rotate around an axis thereof through the second transmission mechanism, so that when the inner cutter tube 22 performs forward rotary cutting for samplings, the inner cutter tube moves forward under the driving of the first motor 11, and meanwhile rotates under the driving of the second motor 12.

In an embodiment, the biopsy sampling device further includes a controller, the controller is electrically connected to the first motor 11 and the second motor 12 respectively, and is configured to obtain a sampling instruction, and control working states of the first motor 11 and the second motor 12 according to the sampling instruction, so as to drive the inner cutter tube 22 to move for samplings, and the samplings include two or more consecutive samplings. The working state includes starting, stopping, a rotation direction, or the like.

The process of driving the inner cutter tube 22 to perform each/single sampling is specifically as follows: firstly, the first motor 11 is controlled to rotate to drive the inner cutter tube 22 to move axially to a specified sampling position (an initial position of the inner cutter tube 22 is usually located at the position where it closes the sampling window, and other positions are also possible), and in the process, the inner cutter tube 22 only moves axially and does not rotate; the specified sampling position corresponds to a preset sampling window opening length, and a sampling length can be adjusted as required; after the inner cutter tube 22 moves to the specified sampling position, the first motor 11 and the second motor 12 are controlled to rotate simultaneously, and the first motor 11 drives the inner cutter tube 22 to move forward through the first transmission mechanism (the rotation direction of the first motor 11 in the forward movement process of the inner cutter tube 22 is opposite to the rotation direction of the first motor 11 in the rearward movement process of the inner cutter tube 22); and meanwhile, the second motor 12 drives the inner cutter tube 22 to rotate around the axis thereof through the second transmission mechanism until the inner cutter tube 22 moves forward to the position where it closes the sampling window, thereby completing one sampling.

The samplings include two or more consecutive/adjacent samplings. During two consecutive/adjacent samplings, the directions of rotation of the inner cutter tube 22 around the axis thereof are opposite. That is, during the two samplings, the directions of rotation of the inner cutter tube 22 around the axis thereof during forward movement are opposite, for example, counterclockwise rotation is performed in the previous sampling, and clockwise rotation is performed in the next sampling.

The double motors are configured in the biopsy handle of the biopsy sampling device, one motor controls the inner cutter tube 22 to move forward and rearward along the axial direction of the outer cutter tube 21, and the other motor controls the inner cutter tube 22 to rotate around the axis of the inner cutter tube. Since the inner cutter tube 22 does not rotate during rearward movement and rotates in different directions in two consecutive samplings during forward movement, a tissue entangled in the previous sampling can be released through reverse rotation in the next sampling, thereby effectively alleviating tissue entanglement, reducing a system fault rate, and improving an operation efficiency.

During puncturing, the inner cutter tube 22 is located at a foremost position to close the sampling window 21a. The inner cutter tube 22 moves rearward to expose the sampling window 21a after reaching the specified position, and the tissue is sucked into the sampling window 21a under negative pressure, at this time, the inner cutter tube 22 moves forward and rotates simultaneously to cut the tissue entering the sampling window 21a, and then, the cut tissue is conveyed to a sample collecting box through the inner cutter tube 22 by a negative pressure device to complete a single sampling operation.

The input instruction at least includes a sampling signal, for example, a button on the handle is pressed to trigger the sampling instruction, or the sampling instruction is input through a host. After the controller receives the sampling instruction, the controller controls the motors to act to start the sampling. One sampling action is performed every time the sampling signal is received. It can also be understood that multiple consecutive sampling actions may be performed every time the sampling signal is received. The specified sampling position may be preset according to the sampling length. For example, typically, the initial position of the inner cutter tube 22 is in the position to close the sampling window, and the inner cutter tube 22 reaches the specified position after moving rearward by a specified distance from the initial position.

Single sampling process: when the biopsy sampling device works, the inner cutter tube 22 moves to the foremost position to close the sampling window 21a before and during the puncturing, and after the puncturing is completed, the inner cutter tube 22 is driven by the first motor 11 to move rearward according to a sampling size requirement until the actual window opening length of the sampling window 21 matches with a set value, the tissue is then sucked into the sampling window 21 by the negative pressure device performing sucking from a rear end of the inner cutter tube 22, and after the action is completed, the inner cutter tube 22 is driven to move forward and rotate at a high speed to cut the tissue and hold the tissue at the front end of the inner cutter tube 22, thereby completing the sampling.

The above controller may be a general-purpose processor, including a central processing unit (CPU), a network processor (NP), or the like. The controller may also be a digital signal processor (DSP), an application specific integrated circuit (ASIC), a field-programmable gate array (FPGA) or other programmable logic devices, discrete gates or transistor logic devices and discrete hardware components. The controller can be arranged on the handle or the host connected to the handle.

In some embodiments, during the two or more adjacent/consecutive samplings, during the previous sampling, the first motor 11 is controlled to rotate to cause the inner cutter tube 22 to move rearward from the position where it closes the sampling window to a first sampling position, and the inner cutter tube 22 only moves axially and does not rotate around the axis thereof in this process. After the inner cutter tube 22 reaches the first sampling position, the first motor 11 and the second motor 12 are controlled to rotate to cause the inner cutter tube 22 to move forward and meanwhile rotate along a first direction until the inner cutter tube 22 reaches the position where it closes the sampling window (that is, returns to the initial position), the tissue entering the sampling window 21a is cut by the inner cutter tube 22, and then, the cut tissue is conveyed to the sample collecting box through the inner cutter tube 22 by virtue of a suction force, thus completing the single sampling operation.

During the next sampling, the first motor 11 is controlled to rotate to cause the inner cutter tube 22 to move rearward to a specified second sampling position, and after the inner cutter tube 22 reaches the second sampling position, the first motor 11 and the second motor 12 are controlled to rotate to cause the inner cutter tube 22 to move forward and rotate along a second direction until the inner cutter tube reaches the position where it closes the sampling window. The first direction is opposite to the second direction. That is, the rotation direction during the forward movement of the inner cutter tube 22 during the previous sampling is opposite to the rotation direction during the forward movement of the inner cutter tube 22 during the next sampling.

The first sampling position and the second sampling position may be the same or different. For example, when the number of samplings is three, the rotation direction of the inner cutter tube 22 in the first sampling is opposite to that in the second sampling, and the rotation direction of the inner cutter tube 22 in the third sampling is opposite to that in the second sampling. The rotation directions in the three samplings are the first direction, the second direction, and the first direction in sequence. Similarly, when the number of samplings is four, the rotation directions in the four samplings are the first direction, the second direction, the first direction and the second direction in sequence. A similar fashion may be adopted as long as the rotation directions in two consecutive samplings are guaranteed to be opposite.

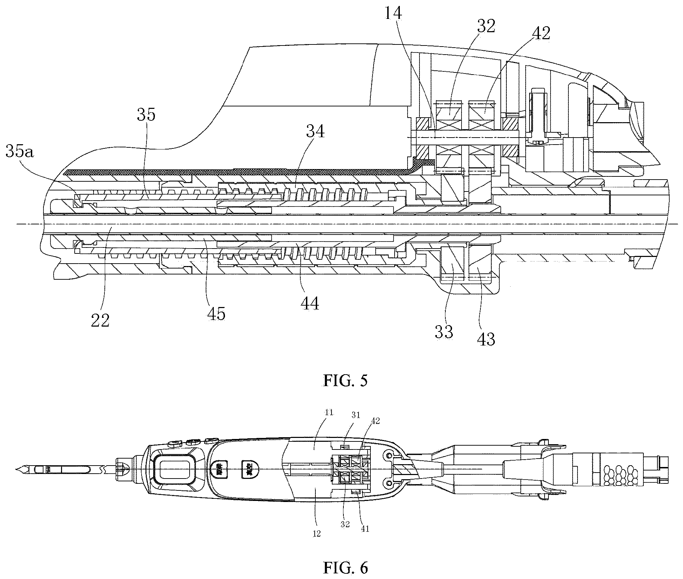

This embodiment exemplifies a transmission manner, and as shown in FIG. 5 to FIG. 6, the first transmission mechanism includes a reciprocating driving gear 31, a reciprocating driven gear 33, a first transmission component 34, and a second transmission component 35. The reciprocating driving gear 31 is coaxially connected to an output end of the first motor 11 and configured to receive the power from the first motor 11. The reciprocating driven gear 33 sleeves the first transmission component 34 and synchronously rotates with the first transmission component 34, and the reciprocating driven gear 33 is meshed with the reciprocating driving gear 31. The first transmission component 34 sleeves the second transmission component 35 and is in threaded fit with the second transmission component 35. The first transmission component 34 only rotates and cannot move along the axial direction of the inner cutter tube 22. The second transmission component 35 and the inner cutter tube 22 are connected in a manner that they are relatively fixed in the axial direction and relatively rotatable in the circumferential direction, that is, the inner cutter tube 22 can rotate around the axis thereof relative to the second transmission component 35, and the second transmission component 35 only moves axially and does not rotate. When the first motor 11 rotates, the first transmission component 34 is driven to rotate around an axis thereof by the reciprocating driving gear 31 and the reciprocating driven gear 33, and since the first transmission component 34 is in threaded fit with the second transmission component 35, the first transmission component 34 does not move axially, and the second transmission component 35 does not rotate circumferentially, the second transmission component 35 can only move axially to drive the inner cutter tube 22 to move axially. The inner cutter tube 22 can be driven to move forward or rearward by changing the rotation direction of the first motor 11.

In some embodiments, as shown in FIG. 5, the biopsy sampling device may further include a housing. The housing being provided with a first limiting structure for limiting the axial movement of the first transmission component 34 along the inner cutter tube 22 and a second limiting structure for limiting the circumferential rotation of the second transmission component 35. The housing can be a housing of the handle, a housing of a cutter or a common housing of the two. The first limiting structure may be a limiting step located outside two axial ends of the first transmission component 34, and the limiting step is located outside the two ends of the first transmission component 34 for blocking. In this embodiment, the first transmission component 34 is a first threaded sleeve with internal threads, the second transmission component 35 is a second threaded sleeve with external threads, and the first threaded sleeve is arranged outside the second threaded sleeve and is coaxial with the inner cutter tube 22. A rear end of the first threaded sleeve has a small-diameter section without threads, and the reciprocating driven gear 33 sleeves the small-diameter section to drive the first threaded sleeve to rotate. The reciprocating driven gear 33 and the first threaded sleeve can be in key-slot fit or fixedly connected. The circumferential rotation of the second transmission component 35 may be limited by a limiting member arranged on the housing, for example, the second transmission component 35 fits the housing via a concave-convex structure.

The inner cutter tube 22 is sleeved with a fourth transmission component 45, and the fourth transmission component 45 is axially and relatively fixedly connected to the inner knife tube 22, and it may rotate synchronously with the inner cutter tube 22 or may not rotate with the inner cutter tube 22. In this embodiment, the fourth transmission component 45 is fixedly connected to the inner cutter tube 22 and axially moves and circumferentially rotates synchronously with the inner cutter tube. An engaging groove 45a is formed in an outer wall of the fourth transmission component 45 along the circumferential direction. The second transmission component 35 sleeves the fourth transmission component 45, an engaging portion 35a extending into the engaging groove 45a is arranged at a front end of the second transmission component 35, and the second transmission component 35 and the fourth transmission component 45 can rotate relatively. When the second transmission component 35 moves back and forth, the fourth transmission component 45 and the inner cutter tube 22 are driven to move axially by the engaging portion 35a and the engaging groove 44a.

In some embodiments, as shown in FIG. 5 to FIG. 7, the second transmission mechanism includes a third transmission component 44, a rotary cutting driving gear 41 and a rotary cutting driven gear 43, the rotary cutting driving gear 41 and the rotary cutting driven gear 43 being meshed with each other. The rotary cutting driving gear 41 is coaxially connected to an output end of the second motor 12. The rotary cutting driven gear 43 sleeves the third transmission component 44 and drives the third transmission component 44 to synchronously rotate. The third transmission component 44 sleeves the inner cutter tube 22, and the third transmission component 44 and the inner cutter tube 22 are fitted in a manner of being axially and relatively movable and circumferentially and relatively fixed.

In an embodiment, the third transmission component 44 may be a transmission sleeve, the transmission sleeve sleeves the inner cutter tube 22, and an inner wall of the transmission sleeve is provided with a groove along the axial direction. The outer cutter tube 21 is fixedly provided with a protrusion portion fitting the groove, the protrusion portion and the groove can move relative to each other in the axial direction, and fitting of the protrusion portion and the groove can drive the inner cutter tube 22 to rotate.

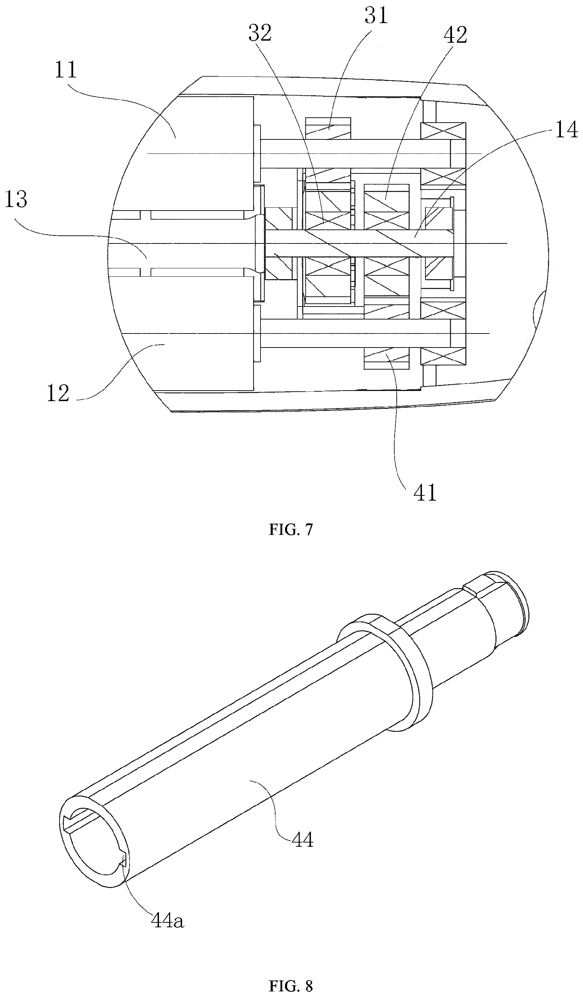

In an embodiment, as shown in FIG. 8 to FIG. 10, the third transmission component 44 is a transmission sleeve. The transmission sleeve is connected to the rotary cutting driven gear 43 and rotates synchronously with the rotary cutting driven gear 43, but the transmission sleeve idly sleeves the inner cutter tube 22, and the transmission sleeve is axially and circumferentially movable relative to the inner cutter tube 22. The fourth transmission component 45 is a connection sleeve. The connection sleeve fixedly sleeves the inner cutter tube 22, a rear portion of the connection sleeve extends into a front portion of the transmission sleeve. The inner wall of the transmission sleeve is provided with a groove 44a along the axial direction. An outer wall of a rear end of the connection sleeve is provided with a corresponding protrusion portion 45a, and the transmission sleeve and the connection sleeve can relatively move axially and be relatively fixed circumferentially. Therefore, in the process that the connection sleeve moves back and forth with the inner cutter tube 22, the connection sleeve and the transmission sleeve transfer torsion through fitting of the protrusion portion 45a and the groove 44a, so as to drive the inner cutter tube 22 to rotate during forward movement. The rotation direction of the inner cutter tube 22 can be adjusted by changing the rotation direction of the second motor 12. The rear portion of the connection sleeve and the front portion of the transmission sleeve are mutually supported, thus further improving coaxiality and transmission stability of the connection sleeve and the transmission sleeve.

A rear portion of the third transmission component 44 is sleeved with the first transmission component 34, and axial movement of the third transmission component 44 may be limited by a shoulder between the third transmission component and the first transmission component 34. The first transmission component 34, the second transmission component 35, the third transmission component 44, and the fourth transmission component 45 may be metal members or injection molded members.

The first transmission mechanism and the second transmission mechanism can also be implemented in other existing ways.

In an embodiment, as shown in FIG. 7, the biopsy sampling device may further include an intermediate shaft 14, as well as a rotary cutting intermediate gear 42 and a reciprocating intermediate gear 32 mounted on the intermediate shaft 14. The first motor 11 and the second motor 12 are arranged side by side on a same bracket 13, the intermediate shaft 14 is mounted on the bracket 13, and the intermediate shaft 14 is parallel to axes of the first motor 11 and the second motor 12 and is located between the first motor 11 and the second motor 12. The rotary cutting intermediate gear 42 is meshed with both the rotary cutting driving gear 41 and the rotary cutting driven gear 43, and the reciprocating intermediate gear 32 is meshed with both the reciprocating driving gear 31 and the reciprocating driven gear 33. This structure facilitates a compact arrangement of the transmission mechanism, thereby reducing a volume of the biopsy sampling device.

An embodiment of the present application further provides a control method for a biopsy sampling device. The control method is applicable to the biopsy sampling device according to any of the above embodiments, and the control method includes: controlling operating states (starting, stopping, and rotation directions) of the first motor 11 and the second motor 12 according to the input sampling instruction to drive the inner cutter tube 22 to move relative to the outer cutter tube 21, so as to perform at least two samplings, where in two consecutive samplings, the directions of rotation of the inner cutter tube 22 around the axis of the inner cutter tube 22 during forward movement are opposite, and the inner cutter tube 22 does not rotate during rearward movement.

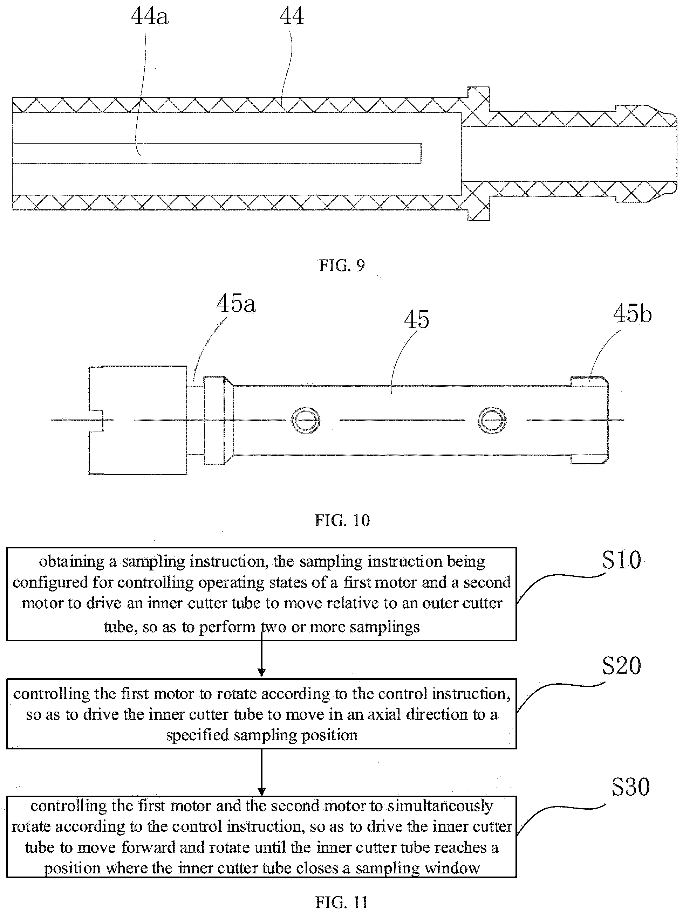

In an embodiment, as shown in FIG. 11, the control method may include the following steps.

In S10, a sampling instruction is obtained, the sampling instruction being configured for controlling operating states of a first motor and a second motor to drive an inner cutter tube to move relative to an outer cutter tube, so as to perform two or more samplings.

In S20, the first motor is controlled to rotate according to the control instruction, so as to drive the inner cutter tube to move in an axial direction to a specified sampling position.

In S30, the first motor and the second motor are controlled to rotate simultaneously according to the control instruction, so as to drive the inner cutter tube to move forward and rotate until the inner cutter tube reaches a position where it closes a sampling window.

The first motor is configured to drive the inner cutter tube to move in the axial direction, and the second motor is configured to drive the inner cutter tube to rotate around an axis of the inner cutter tube. The inner cutter tube rotates in opposite directions around the axis of the inner cutter tube in two consecutive samplings.

For ease of understanding, steps of the above control method involving different sampling times are described in detail below by way of specific examples.

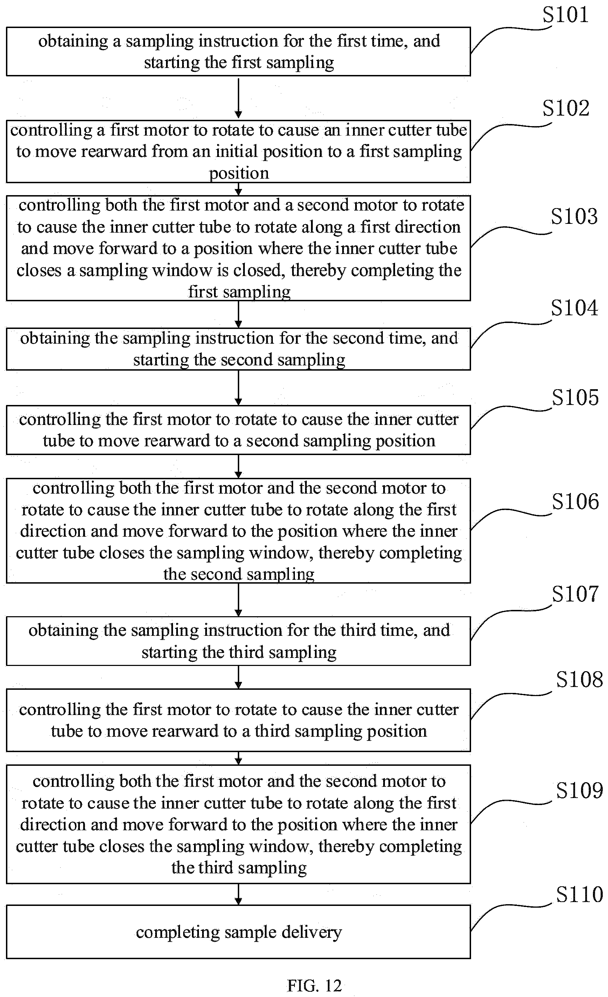

In an embodiment, as an example shown in FIG. 12, for three consecutive samplings, a specific process of the control method includes the following steps.

In S101, a sampling instruction is obtained for the first time or a first sampling instruction is obtained, and the first sampling is started.

In S102, a first motor 11 is controlled to rotate to cause an inner cutter tube 22 to move rearward to a first sampling position.

Specifically, the first motor 11 is controlled to rotate to cause the inner cutter tube 22 to move rearward by a specified length or distance to the specified first sampling position. The specified first sampling position may be set or input by a host, or the like, before the step S101. The inner cutter tube 22 does not rotate during the rearward movement, that is, a second motor 12 does not rotate.

In S103, the first motor 11 is controlled to rotate to cause the inner cutter tube 22 to move forward, and meanwhile the second motor 12 is controlled to rotate to cause the inner cutter tube 22 to rotate in a first direction (e.g., forward or clockwise) until the inner cutter tube 22 reaches a position where it closes a sampling window, thereby completing the first sampling.

In S104, the sampling instruction is obtained for the second time or a second sampling instruction is obtained, and the second sampling is started.

In S105, the first motor 11 is controlled to rotate to cause the inner cutter tube 22 to move rearward to a second sampling position.

In S106, the first motor 11 is controlled to rotate to cause the inner cutter tube 22 to move forward, and meanwhile the second motor 12 is controlled to rotate to cause the inner cutter tube 22 to rotate in a second direction (e.g., rearward or counterclockwise) until the inner cutter tube 22 reaches the position where it closes the sampling window, thereby completing the second sampling, where a rotation direction of the second motor 12 in this step is opposite to that of the second motor 12 in S103.

In S107, the sampling instruction is obtained for the third time or a third sampling instruction is obtained, and the third sampling is started.

In S108, the first motor 11 is controlled to rotate to cause the inner cutter tube 22 to move rearward to a specified third sampling position.

In S109, the first motor 11 is controlled to rotate to cause the inner cutter tube 22 to move forward, and meanwhile the second motor 12 is controlled to rotate to cause the inner cutter tube 22 to rotate in the first direction (e.g., forward or clockwise) until the inner cutter tube 22 reaches the position where it closes the sampling window, thereby completing the third sampling, where the rotation direction of the second motor 12 in this step is opposite to that of the second motor 12 in S106.

In S110, after the multiple samplings are finished, all samples are delivered through negative pressure suction to complete sample delivery.

The sampling instruction can be triggered by pressing a key on a handle or input through the host. For example, the sampling instruction is input once every time the key of the handle is pressed.

Two or more samplings are sequentially performed in this way, and the first sampling position, the second sampling position and the third sampling position can be the same or different. For example, when the number of samplings is three, the rotation direction of the inner cutter tube 22 in the first sampling is opposite to that in the second sampling, and the rotation direction of the inner cutter tube 22 in the third sampling is opposite to that in the second sampling: the rotation directions of the inner cutter tube 22 in the three samplings are the first direction, the second direction, and the first direction in sequence. Similarly, when the number of samplings is four, the rotation directions of the inner cutter tube 22 in the four samplings are the first direction, the second direction, the first direction and the second direction in sequence. A similar fashion may be adopted as long as the rotation directions of the inner cutter tube 22 in two consecutive samplings are guaranteed to be opposite.

Before the first sampling is initiated, self-checking of the biopsy sampling device, setting of a sampling length, and positioning of the inner cutter tube 22 at an initial position (the position where the inner cutter tube closes the sampling window), or the like, may be performed.

In the above embodiment, by inputting the first sampling instruction (pressing the sampling key once), the entire action of moving the inner cutter tube 22 backward to the specified sampling position and rotating and moving the inner cutter tube forward to the position where it closes the sampling window is performed.

In another embodiment, rearward movement of the inner cutter tube 22 requires the corresponding sampling instruction, and rotation and forward movement of the inner cutter tube 22 require another sampling instruction. In other words, during each sampling, when the first sampling instruction is input/obtained, the inner cutter tube 22 moves rearward to the specified position, and when the second sampling instruction is input/obtained, the inner cutter tube 22 rotates and meanwhile moves forward to the position where it closes the sampling window. Each input of the sampling instruction may be realized by pressing the sampling key, and distinguishing may be realized by long pressing and short pressing. Distinguishing may alternatively be realized by a pressing sequence, for example, the inner cutter tube 22 moves rearward during the first pressing, rotates and moves forward during the second pressing, again moves rearward during the third pressing, and rotates and moves forward during the fourth pressing. It should be noted that one complete sampling process can be performed by inputting/obtaining the sampling instruction once, for example, pressing the key for a long time. Alternatively, multiple consecutive samplings are performed by inputting/obtaining the sampling instruction once.

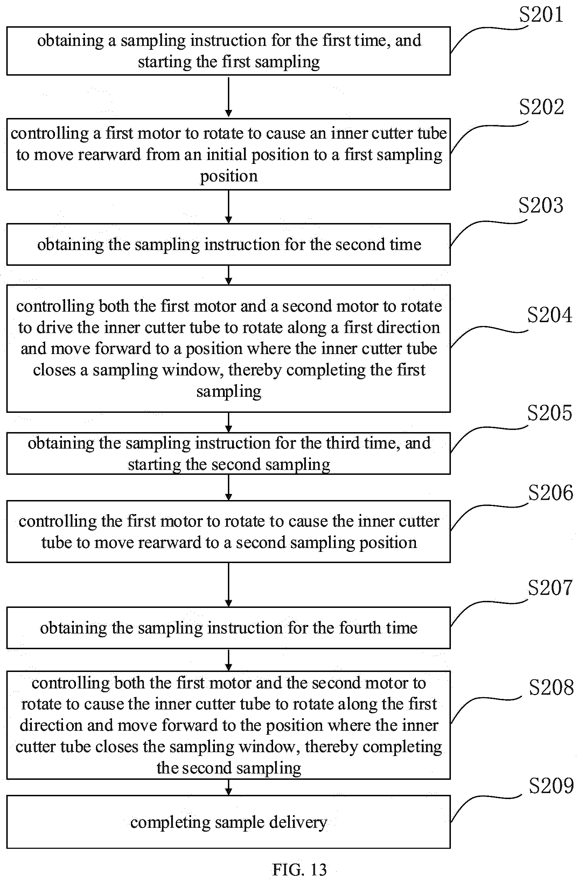

In an embodiment, as an example shown in FIG. 13, for two consecutive samplings, a specific process of the control method includes the following steps.

In S201, a sampling instruction is obtained for the first time or a first sampling instruction is obtained, and the first sampling is started. For example, the sampling instruction is input by pressing a key of a handle.

In S202: a first motor 11 is controlled to rotate to cause an inner cutter tube 22 to move rearward to a first sampling position, and meanwhile the inner cutter tube 22 is controlled not to rotate during rearward movement, that is, that is, a second motor 12 is controlled not to rotate.

In S203, the sampling instruction is obtained for the second time or a second sampling instruction is obtained. The second time of inputting of the sampling instruction may be the second time of pressing of the key of the handle.

In S204, the first motor 11 is controlled to rotate to cause the inner cutter tube 22 to move forward, and meanwhile the second motor 12 is controlled to rotate to cause the inner cutter tube 22 to rotate in a first direction (e.g., forward or clockwise) until the inner cutter tube 22 reaches a position where it closes a sampling window, thereby completing the first sampling.

In S205, the sampling instruction is obtained for the third time or a third sampling instruction is obtained, and the second sampling is started. The third sampling instruction may be input by pressing the key of the handle for the third time.

In S206, the first motor 11 is controlled to rotate to cause the inner cutter tube 22 to move rearward to a second sampling position.

In S207, the sampling instruction is obtained for the fourth time or a fourth sampling instruction is obtained. The input of the sampling instruction can be triggered by pressing the key of the handle for the fourth time.

In S208, the first motor 11 is controlled to rotate to cause the inner cutter tube 22 to move forward, and meanwhile the second motor 12 is controlled to rotate to cause the inner cutter tube 22 to rotate in a second direction (e.g., rearward or counterclockwise) until the inner cutter tube 22 reaches the position where it closes the sampling window, thereby completing the second sampling.

In S209, after the two samplings are finished, all samples are delivered through negative pressure suction to complete sample delivery.

In another embodiment, the sample may be delivered after each sampling is completed, and not delivered collectively at the end.

The implementation of the above control method is not dependent on the biopsy sampling device according to this embodiment, and the control method can also be applied to other double-motor driven biopsy sampling devices, as long as one motor drives the inner cutter tube to move back and forth, and the other motor drives the inner cutter tube to rotate around the axis of the inner cutter tube.

In the present application, the double motors are configured in the biopsy handle of the biopsy sampling device, one motor is configured to control the inner cutter tube 22 to move forward and rearward along the axial direction of the outer cutter tube 21, the other motor is configured to control the inner cutter tube 22 to rotate during forward movement, the inner cutter tube 22 does not rotate during rearward movement, and the inner cutter tube rotates along a different direction during forward movement in each sampling. In this way, a tissue entangled in the previous sampling can be released through reverse rotation in the next sampling, thereby effectively alleviating tissue entanglement, reducing a system fault rate, and improving an operation efficiency.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the specification.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the protection scope of the present application should be subject to the appended claims.

## Claims

1. A control method for a biopsy sampling device, the biopsy sampling device comprising an outer cutter tube and an inner cutter tube, the outer cutter tube sleeving the inner cutter tube, a sampling window being arranged in a side of a front end of the outer cutter tube, and the control method comprising:
obtaining a sampling instruction, the sampling instruction being configured for controlling operating states of a first motor and a second motor to drive the inner cutter tube to move relative to the outer cutter tube, so as to perform two or more samplings;
controlling the first motor to rotate according to the control instruction, so as to drive the inner cutter tube to move in an axial direction to a specified sampling position; and
controlling the first motor and the second motor to simultaneously rotate according to the control instruction, so as to drive the inner cutter tube to move forward and rotate until the inner cutter tube reaches a position where the inner cutter tube closes the sampling window;
wherein the first motor is configured to drive the inner cutter tube to move in the axial direction, and the second motor is configured to drive the inner cutter tube to rotate around the axis of the inner cutter tube; directions of rotation of the inner cutter tube around the axis of the inner cutter tube are opposite in two consecutive samplings.

2. The control method for a biopsy sampling device according to claim 1, further comprising: during the two consecutive samplings,
during the previous sampling, controlling the first motor to rotate to cause the inner cutter tube to move rearward from the position where the inner cutter tube closes the sampling window to a first sampling position, and after the inner cutter tube reaches the first sampling position, controlling the first motor and the second motor to rotate to cause the inner cutter tube to move forward and meanwhile rotate along a first direction until the inner cutter tube reaches the position where the inner cutter tube closes the sampling window;
during the next sampling, controlling the first motor to rotate to cause the inner cutter tube to move rearward to a specified second sampling position, and after the inner cutter tube reaches the second sampling position, controlling the first motor and the second motor to rotate to cause the inner cutter tube to move forward and rotate along a second direction until the inner cutter tube reaches the closed position of the sampling window;
wherein the first direction is opposite to the second direction, and rotation directions of the second motor during the two consecutive samplings are opposite.

3. The control method for a biopsy sampling device according to claim 1 or 2, wherein one sampling is performed every time the sampling instruction is received; or two or more consecutive samplings are performed every time the sampling instruction is received.

4. The control method for a biopsy sampling device according to claim 1 or 2, wherein during each sampling, when a first sampling instruction is received, the first motor is controlled to rotate to cause the inner cutter tube to move rearward to the specified first sampling position; and after the inner cutter tube reaches the first sampling position and a second sampling instruction is received, the first motor and the second motor are controlled to rotate simultaneously to cause the inner cutter tube to move forward and rotate along the second direction until the inner cutter tube reaches the position where the inner cutter tube closes the sampling window.

5. A biopsy sampling device, comprising an outer cutter tube and an inner cutter tube, the outer cutter tube sleeving the inner cutter tube, a sampling window being arranged in a side of a front end of the outer cutter tube, and the biopsy sampling device further comprising:
a first motor, the first motor being connected to the inner cutter tube through a first transmission mechanism to drive the inner cutter tube to move along an axial direction;
a second motor, the second motor being connected to the inner cutter tube through a second transmission mechanism to drive the inner cutter tube to rotate around an axis of the inner cutter tube; and
a controller respectively connected to the first motor and the second motor and configured to obtain a sampling instruction and control operating states of the first motor and the second motor according to the sampling instruction to drive the inner cutter tube to move relative to the outer cutter tube, so as to perform two or more samplings;
wherein the controller is further configured to:
control the first motor to rotate according to the sampling instruction, so as to drive the inner cutter tube to move in the axial direction to a specified sampling position; and
control the first motor and the second motor to simultaneously rotate according to the sampling instruction, so as to drive the inner cutter tube to move forward and rotate until the inner cutter tube reaches a position where the inner cutter tube closes the sampling window, thereby completing the sampling;
wherein the first motor is configured to drive the inner cutter tube to move in the axial direction, and the second motor is configured to drive the inner cutter tube to rotate around the axis of the inner cutter tube; during two consecutive samplings, directions of rotation of the inner cutter tube around the axis of the inner cutter tube are opposite.

6. The biopsy sampling device according to claim 5, wherein the first transmission mechanism comprises a reciprocating driving gear and a reciprocating driven gear that are meshed with each other, the first transmission mechanism further comprises a first transmission component and a second transmission component that are sleeved by threads, the reciprocating driving gear being connected to an output end of the first motor, the reciprocating driven gear being mounted on the first transmission component and driving the first transmission component to rotate synchronously, and the second transmission component and the inner cutter tube being connected in a mode of being axially relatively fixed and circumferentially relatively rotatable.

7. The biopsy sampling device according to claim 6, wherein the biopsy sampling device further comprises a handle housing, a first limiting structure for limiting axial movement of the first transmission component and a second limiting structure for limiting circumferential rotation of the second transmission component being arranged on the handle housing.

8. The biopsy sampling device according to claim 6, wherein a fourth transmission component sleeves the inner cutter tube, the fourth transmission component is axially and relatively fixedly connected to the inner cutter tube, an engaging groove is formed in an outer wall of the fourth transmission component along a circumferential direction, the second transmission component sleeves the fourth transmission component, an engaging portion extending into the engaging groove is formed in the second transmission component, and the second transmission component drives the fourth transmission component and the inner cutter tube to move axially through the engaging portion and the engaging groove.

9. The biopsy sampling device according to any one of claims 5 to 8, wherein the second transmission mechanism comprises a third transmission component, a rotary cutting driving gear, and a rotary cutting driven gear, the rotary cutting driving gear and the rotary cutting driven gear being meshed with each other, the rotary cutting driving gear is connected to an output end of the second motor, the rotary cutting driven gear is mounted on the third transmission component and drives the third component to rotate synchronously, the third transmission component sleeves the inner cutter tube, and the third transmission component and the inner cutter tube are fitted in a mode of being axially and relatively movable and circumferentially and relatively fixed.

10. The biopsy sampling device according to claim 9, wherein the third transmission component sleeves the inner cutter tube, a groove is formed in an inner wall of the third transmission component along the axial direction, a protrusion portion fitting the groove is fixedly arranged on the outer cutter tube, and the protrusion portion and the groove are capable of moving relative to each other in the axial direction.

11. The biopsy sampling device according to claim 9, further comprising an intermediate shaft, a rotary cutting intermediate gear, and a reciprocating intermediate gear, the rotary cutting intermediate gear and the reciprocating intermediate gear being mounted on the intermediate shaft, the first motor and the second motor being arranged side by side, the intermediate shaft being arranged in parallel between the first motor and the second motor, the rotary cutting intermediate gear being meshed with the rotary cutting driving gear and the rotary cutting driven gear simultaneously, and the reciprocating intermediate gear being meshed with the reciprocating driving gear and the reciprocating driven gear simultaneously.
